(19) Europäisches Patentamt
European Patent Office
Office européen des brevets

(11) **EP 4 585 218 A1**

(12) **EUROPEAN PATENT APPLICATION**

(43) Date of publication:
**16.07.2025 Bulletin 2025/29**

(21) Application number: **25151303.2**

(22) Date of filing: **10.01.2025**

(51) International Patent Classification (IPC):
**A61K 33/00** (2006.01)    **A61K 33/24** (2019.01)
**A61P 1/00** (2006.01)    **A61P 9/10** (2006.01)
**A61P 9/12** (2006.01)    **A61P 29/00** (2006.01)

(52) Cooperative Patent Classification (CPC):
**A61K 33/00; A61K 33/24; A61P 1/00; A61P 9/10;
A61P 9/12; A61P 29/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **12.01.2024 JP 2024003230**

(71) Applicant: **Murata Manufacturing Co., Ltd.
Nagaokakyo-shi, Kyoto 617-8555 (JP)**

(72) Inventors:
• **KIMURA, Yuki**
 **Nagaokakyo-shi, Kyoto, 617-8555 (JP)**
• **MEGUMI, Daisuke**
 **Nagaokakyo-shi, Kyoto, 617-8555 (JP)**
• **TAKASE, Hajime**
 **Yokohama-shi, Kanagawa, 236-0004 (JP)**
• **KOBAYASHI, Yusuke**
 **Yokohama-shi, Kanagawa, 236-0004 (JP)**

(74) Representative: **Behr, Wolfgang
Lorenz Seidler Gossel
Rechtsanwälte Patentanwälte
Partnerschaft mbB
Widenmayerstraße 23
80538 München (DE)**

(54) **PHARMACEUTICAL COMPOSITION FOR IMPROVING INTESTINAL BACTERIAL FLORA**

(57)    One object of the present disclosure is to provide a novel pharmaceutical composition, preferably a pharmaceutical composition capable of improving the intestinal bacterial flora in vivo or increasing a short-chain fatty acid in vivo.

    A pharmaceutical composition comprising MXene for improving intestinal bacterial flora in vivo or increasing a short-chain fatty acid in vivo.

EP 4 585 218 A1

**Description**

TECHNICAL FIELD

The present disclosure relates to a pharmaceutical composition, a method for improving an intestinal bacterial flora, a treatment method, and a prevention method.

BACKGROUND ART:

In recent years, the relevance between the intestinal environment and various diseases has been suggested, and the improvement of the intestinal environment is expected to have the treatment effect of these diseases.

Patent Document 1 describes a pharmaceutical composition for improving the intestinal bacterial flora, containing 1-cyclopropyl-6 fluoro-1,4-dihydro-8 methyl-7-(2-amino-3-cyano-5-pyridyl)-4-oxo-3-quinoline carboxylic acid or a pharmaceutically acceptable salt thereof as an active ingredient.

Non Patent Document 1 describes that by administering genetically engineered E. coli to dextran sulfate sodium-induced colitis (DSS) mice as a model animal of inflammatory bowel disease (IBD), the diversity of intestinal bacterial flora is promoted to proliferate the number of Ruminococcaceae, and as a result, the production of Butyric acid can be enhanced.

Non Patent Document 2 describes that diversity of intestinal bacteria can promote expression of Cldn and reduce the permeability of Blood Brain Barrier (BBB).

Non Patent Document 3 describes that Butyrate can dominantly correct the hypertensive symptoms of a pregnant female.

Patent Document

Patent Document 1: JP 2023-12558 A

Non Patent Documents

Non Patent Document 1: Lifu Wang et al., An engineered probiotic secreting Sj16 ameliorates colitis via Ruminococcaceae/butyrate/retinoic acid axis, BIOENGINEERING & TRANSLATIONAL MEDICINE, volume 6, Issue 3 September 2021

Non Patent Document 2: V. Braniste et al., The gut microbiota influences blood-brain barrier permeability in mice, 2014, Science Translational Meddicine, 6: 263ra158

Non Patent Document 3: Luisa F. Gomez-Arango et al., Increased Systolic and Diastolic Blood Pressure Is Associated With Altered Gut Microbiota Composition and Butyrate Production in Early Pregnancy, Hyperteinsion October 2016 Vol 68, Issue 4

SUMMARY

Problems to be Solved

Patent Document 1 describes that an intestinal bacterial flora can be improved by administering a predetermined compound, and Non Patent Documents 1 to 3 describes that when E. coli is directly administered, it may proliferate Ruminococcaceae, which is a kind of intestinal bacteria, and Butyric acid, which may be produced thereby, will contribute to the suppression of permeability enhancement in Blood Brain Barrier (BBB) and amelioration of hypertensive symptoms. However, there is no description about the action of MXene.

One object of the present disclosure is to provide a novel pharmaceutical composition, preferably a pharmaceutical composition capable of improving the intestinal bacterial flora in vivo or increasing a short-chain fatty acid in vivo.

Solutions

The pharmaceutical composition of the present disclosure comprises MXene and is used for improving the intestinal bacterial flora in vivo.

Effects

The present disclosure will provide a novel pharmaceutical composition, and preferably will provide a pharmaceutical composition capable of improving the intestinal bacterial flora in vivo or increasing a short-chain fatty acid in vivo. The present disclosure may also provide a treatment method or prevention method.

The pharmaceutical composition of the present disclosure comprises MXene, can promote the proliferation of intestinal bacteria, can improve the intestinal bacterial flora, or can increase a short-chain fatty acid in vivo. Therefore, it is useful for the treatment and/or prevention of various diseases.

Although not to be construed as being limited to a specific theory, MXene comprised in the pharmaceutical composition of the present disclosure is considered to have the ability to promote the proliferation of intestinal bacteria in the intestine, and the actual promotion of the bacteria growth is expected. In addition, the proliferation of a short-chain fatty acid, which can be produced by metabolism of these intestinal bacteria, is also expected to be promoted by the proliferation of these intestinal bacteria. As a result, the action of the short-chain fatty acid is expected to promote the repair of the blood vessel barrier. The intake of MXene is also expected to lower the blood pressure. Since MXene is not considered to be absorbed from the intestinal tract, MXene is expected to pass through the gastrointestinal tract and be excreted as it is together with feces. As described above, although a living body originally has two excretion functions of bile excretion and urine excretion, the metabolic pathway by the pharmaceutical composition of the present disclosure can also be interpreted to be a third metabolic pathway, and is expected to lead to the reduction of treatment related to dialysis therapy in patients with renal failure, for example. In addition, MXene of the present disclosure is also expected to adsorb disease causing substances and the like, which are contained in the contents of the diet in the gastrointestinal tract and does not cause intestinal tract absorption.

BRIEF DESCRIPTION OF DRAWINGS

Figs. 1(a) and 1(b) are schematic cross-sectional views showing MXene particles of a layered material in one embodiment of the present disclosure, in which Fig. 1(a) shows single-layer MXene particles, and Fig. 1(b) shows multilayer (exemplarily, two layers) MXene particles;

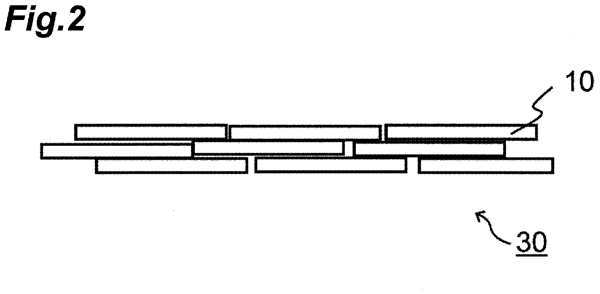

Fig. 2 is a schematic cross-sectional view showing a material in one embodiment of the present disclosure;

Figs. 3(a) and 3(b) show the results of a MXene administration test to a hypertensive mouse, in which Fig. 3(a) shows a change in systolic blood pressure (SBP), and Fig. 3(b) shows a change in diastolic blood pressure (DBP);

Fig. 4 shows a result of a MXene administration test to a hypertensive mouse, and shows a behavior area of a mouse in cognitive behavior analysis;

Fig. 5 shows a result of a MXene administration test to a hypertensive mouse, and shows a Time of Entries Discrimination Index in cognitive behavior analysis;

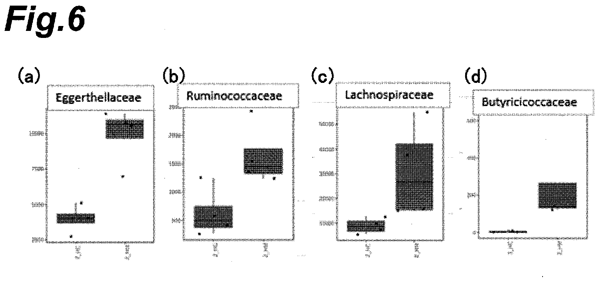

Figs. 6(a) to 6(d) show the results of the MXene administration test to a hypertensive mouse, and in 16s-rRNA analysis, Fig. 6(a) shows the abundance ratio of family Eggerthellaceae in order Coriobacteriales of class Coriobacteriia of phylum Actinobacteriota; Fig. 6(b) shows the abundance ratio of family Ruminococcaceae in order Oscillospirales of class Clostridia of phylum Firmicutes; Fig. 6(c) shows the abundance ratio of family Lachnospirales of class Clostridia of phylum Firmicutes of order Lachnospiraceae; and Fig. 6(d) shows the abundance ratio of family Butyricicoccaceae in order Oscillospirales of class Clostridia of phylum Firmicutes;

Figs. 7(a) and 7(b) show the results of the MXene administration test to a hypertensive mouse, and in 16s-rRNA analysis, Fig. 7(a) shows $\alpha$ diversity of intestinal bacterial flora, and Fig. 7(b) shows $\beta$ diversity drawn by PCoA;

Figs. 8(a) to 8(c) show the results of a MXene administration test to a hypertensive mouse, and show changes in the amounts of Fig. 8(a) butyric acid, Fig. 8(b) acetic acid, and Fig. 8(c) propionic acid in CE-MS;

Fig. 9 shows the results of a test of MXene administration to a hypertensive mouse, and shows a change in Cldn5 in RNA seq of a callosum cell;

Fig. 10 shows the result of the MXene administration test to a hypertensive mouse, and shows a fluorescence microscopic observation image stained with myelin; and

Figs. 11(a) and 11(b) show the results of the MXene administration test to a hypertensive mouse, and show the amount of MBP measured by the WB method, where the MBP amount Fig. 11(a) is a photograph of the membrane after transfer, and Fig. 11(b) shows a ratio of the amount of MBP based on the amount of $\beta$-actin.

DETAILED DESCRIPTION

The pharmaceutical composition of the present disclosure comprises Mxene. Mxene is typically a layered material having the form of one or more layers. In general, MXene has the form of particles of such a layered material (may comprise powders, flakes, nanosheets, etc.).

The Mxene preferably comprises two-dimensional particles of a layered material having one or more layers. The layer preferably comprises at least one metal selected from group 3, 4, 5, 6, and 7 metals and at least one selected from a carbon atom and a nitrogen atom.

The group 3, 4, 5, 6, and 7 metals is preferably at least one selected from the group consisting of Ti, Zr, Hf, V, Nb, Ta, Cr, Mo, Sc, W, and Mn, and more preferably at least one selected from the group consisting of Ti, V, Cr, and Mo.

The layer preferably comprises a layer body represented by the following composition formula:

$$M_m X_n$$

wherein M is at least one group 3, 4, 5, 6, and 7 metals,

X is a carbon atom, a nitrogen atom, or a combination thereof,
n is 1 to 4, and
m is more than n and 5 or less.

It is preferred that the layer further comprises a modification or termination T present on a surface of the layer body in which T is at least one selected from the group consisting of a hydroxyl group, a fluorine atom, a chlorine atom, an oxygen atom, and a hydrogen atom.

The pharmaceutical composition of the present disclosure comprises Mxene, preferably two-dimensional particles having a layer body represented by $M_m X_n$ and T, and can adsorb a substance that can cause a disease (disease causing substance), and thus is useful for the treatment and/or prevention of various diseases.

In the present disclosure, the layered material may be understood as a layered compound, and the layer is also referred to as "$M_m X_n T_s$". In the formula, s is an arbitrary number, and conventionally, x or z may be used instead of s. Hereinafter, the layered material may be referred to as MXene, the layer may be referred to as MXene layer, and the two-dimensional particles may be referred to as MXene two-dimensional particles or MXene particles.

In the present disclosure, when an element is referred to as an "atom", the oxidation number of the element is not limited to 0, and may be an arbitrary number within the range of possible oxidation numbers of the element.

In addition, with respect to the reference numerals of the general formulae shown in the present disclosure, unless otherwise specified, definitions for the same reference numerals are common among the general formulae including the reference numerals.

In the above formula: $M_m X_n$, m may typically be, but not limited to, 2, 3, 4, or 5. Also, n may be, but is not limited to, 1, 2, 3, or 4. In one aspect, m may be 3 and n may be 2.

In the above formula: $M_m X_n$, M is preferably at least one selected from the group consisting of Ti, Zr, Hf, V, Nb, Ta, Cr, Mo, Sc, W, and Mn, and more preferably at least one selected from the group consisting of Ti, V, Cr, and Mo.

As $M_m X_n$, one expressed as follows is known.

$Sc_2C$, $Ti_2C$, $Ti_2N$, $Zr_2C$, $Zr_2N$, $Hf_2C$, $Hf_2N$, $V_2C$, $V_2N$, $Nb_2C$, $Ta_2C$, $Cr_2C$, $Cr_2N$, $Mo_2C$, $Mo_{1.3}C$, $Cr_{1.3}C$, $(Ti, V)_2C$, $(Ti, Nb)_2C$, $W_2C$, $W_{1.3}C$, $Mo_2N$, $Nb_{1.3}C$, $Mo_{1.3}Y_{0.6}C$ (In the above formula, "1.3" and "0.6" mean about 1.3 (= 4/3) and about 0.6 (= 2/3), respectively.),
$Ti_3C_2$, $Ti_3N_2$, $Ti_3$ (CN), $Zr_3C_2$, $(Ti, V)_3C_2$, $(Ti_2Nb)C_2$, $(Ti_2Ta)C_2$, $(Ti_2Mn)C_2$, $Hf_3C_2$, $(Hf_2V)C_2$, $(Hf_2Mn)C_2$, $(V_2Ti)C_2$, $(Cr_2Ti)C_2$, $(Cr_2V)C_2$, $(Cr_2Nb)C_2$, $(Cr_2Ta)C_2$, $(Mo_2Sc)C_2$, $(Mo_2Ti)C_2$, $(Mo_2Zr)C_2$, $(Mo_2Hf)C_2$, $(Mo_2V)C_2$, $(Mo_2Nb)C_2$, $(Mo_2Ta)C_2$, $(W_2Ti)C_2$, $(W_2Zr)C_2$, $(W_2Hf)C_2$,
$Ti_4N_3$, $V_4C_3$, $Nb_4C_3$, $Ta_4C_3$, $(Ti, Nb)_4C_3$, $(Nb, Zr)_4C_3$, $(Ti_2Nb_2)C_3$, $(Ti_2Ta_2)C_3$, $(V_2Ti_2)C_3$, $(V_2Nb_2)C_3$, $(V_2Ta_2)C_3$, $(Nb_2Ta_2)C_3$, $(Cr_2Ti_2)C_3$, $(Cr_2V_2)C_3$, $(Cr_2Nb_2)C_3$, $(Cr_2Ta_2)C_3$, $(Mo_2Ti_2)C_3$, $(Mo_2Zr_2)C_3$, $(Mo_2Hf_2)C_3$, $(Mo_2V_2)C_3$, $(Mo_2Nb_2)C_3$, $(Mo_2Ta_2)C_3$, $(W_2Ti_2)C_3$, $(W_2Zr_2)C_3$, $(W_2Hf_2)C_3$, $(Mo_{2.7}V_{1.3})C_3$ (In the above formula, "2.7" and "1.3" mean about 2.7 (= 8/3) and about 1.3 (= 4/3), respectively.)

Typically, in the above formula: $M_m X_n$, M may be Ti or V, X may be a carbon atom or a nitrogen atom, M may be Ti, and X may be a carbon atom. In one aspect, MXene may be $Ti_3C_2T_s$ (In other words, M is Ti, X is C, n is 2, and m is 3.). In this case, a precursor of such MXene (also referred to as a "MAX phase") may be $Ti_3AlC_2$.

MXene can be produced by removing A atoms comprised in the MAX phase of the precursor (In one aspect, it is represented by $M_m AX_n$, M, m, X, and n have the same meaning as described above, and A is at least one group 12, 13, 14, 15, or 16 element.), and MXene may comprise such A atoms. In one aspect, the residual amount of A atoms comprised in MXene can be preferably 10% by mass or less, more preferably 8% by mass or less, and still more preferably 6% by mass or less with respect to the content of A atoms in the precursor.

In another aspect, the residual amount of A atoms may be more than 10% by mass. For example, those in which the A atom is removed from only a part of the MAX phase are also comprised in the technical scope of the MXene.

Examples of such MXene include MXene in which A atoms are removed only from the vicinity of the end in the plane direction of the MAX phase (direction parallel to the plane of the $M_mX_n$ layer comprised in the MAX phase). In this aspect, the residual amount of the A atoms may be, for example, 50% by mass or more, further 80% by mass or more, and particularly 90% by mass or more.

The content of lithium in the MXene is preferably 0% by mass to 0.1% by mass, more preferably 0% by mass to 0.01% by mass, and still more preferably 0% by mass to 0.002% by mass. When the content of lithium is within the above range, biocompatibility can be improved.

The content of lithium in MXene can be measured by inductively coupled plasma atomic emission spectrometry (ICP-AES).

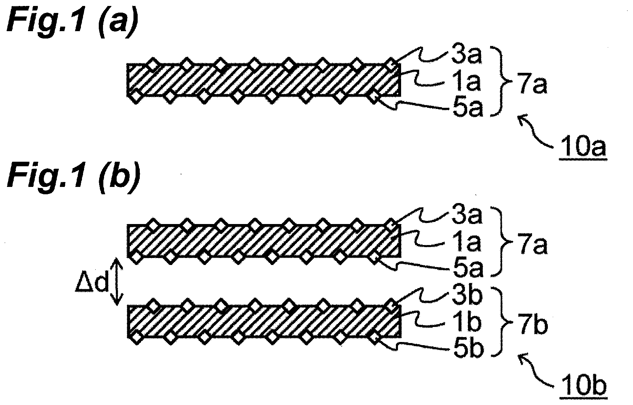

The MXene is an aggregate comprising MXene particles (hereinafter, simply referred to as "MXene particles") 10a (single-layer MXene particles) of one layer schematically exemplified in Fig. 1(a). Typically, the MXene particle 10a is an MXene layer 7a having a layer body ($M_mX_n$ layer) 1a represented by $M_mX_n$ and modifications or terminations T 3a, 5a present on the surface of the layer body 1a (More specifically, at least one of two surfaces facing each other in each layer). Therefore, the MXene layer 7a is also represented as "$M_mX_nT_s$", and s is an arbitrary number.

The MXene may comprise one or more layers. Examples of the MXene particles (multilayer MXene particles) of the plurality of layers include, but are not limited to, the MXene particle 10b of two layers as schematically shown in Fig. 1(b). 1b, 3b, 5b, and 7b in Fig. 1(b) are the same as 1a, 3a, 5a, and 7a in Fig. 1(a) described above. Two adjacent MXene layers (for example, 7a and 7b) of the multilayer MXene particles may not necessarily be completely separated from each other, but may be partially in contact with each other. In the single-layer MXene particle 10a, the multilayer MXene particle 10b are individually separated and exist in one layer. The MXene may be a mixture of the single-layer MXene particle 10a and the multilayer MXene particle 10b in which unseparated multilayer MXene particle 10b remain.

Typically, at least one of the surfaces of the layer body 1a represented by $M_mX_n$ can be planar (two-dimensional), and all of the surfaces of the layer body 1a can be planar (two-dimensional).

Although the present embodiment is not limited, the thickness of each layer (corresponds to the MXene layers 7a and 7b) comprised in the MXene particles is, for example, 0.8 nm to 5 nm, particularly 0.8 nm to 3 nm (It may vary mainly depending on the number of M atom layers comprised in each layer.).

The thickness of each layer is determined as a number average dimension (for example, a number average of at least 40) based on an atomic force microscope (AFM) photograph or a transmission electron microscope (TEM) photograph.

For each laminate of MXene (particularly multilayer MXene particles that may be comprised), the interlayer distance (Alternatively, the void dimension is indicated by Δd in Fig. 1(b).) may be, for example, 0.8 nm or more and 10 nm or less, particularly 0.8 nm to 5 nm, and more particularly about 1 nm, and the total number of layers may be 2 to 20,000.

The interlayer distance in MXene can be measured by obtaining an interplanar distance (the sum of the interlayer distance and the thickness of each layer) from the position of a peak corresponding to the (002) plane of MXene present at $2\theta = 10°$ (deg) or less in X-ray diffraction measurement of MXene and subtracting the thickness of each layer from the interplanar distance.

The MXene may comprise MXene particles having a small number of layers. The "small number of layers" means that, for example, the number of laminated MXene layers is six or less. In addition, the thickness of the multilayer MXene particles having a small number of layers in the lamination direction is preferably 15 nm or less, and more preferably 10 nm or less. Hereinafter, the "multilayer MXene particles having a small number of layers" may be referred to as "few-layer MXene particles". The single-layer MXene particles and the few-layer MXene particles may be collectively referred to as "single-layer/few-layer MXene particles".

In the MXene, the ratio of the single-layer/few-layer MXene particles having a thickness of 15 nm or less may be 0 vol% to 100 vol%, further 0 vol% to 99 vol%, still further 0 vol% to 50 vol%, and particularly 0 vol% to 30 vol%.

(Average value of major axes of two-dimensional surface of MXene)

The major axis of MXene is preferably 1 μm to 20 μm in a plane (hereinafter, also referred to as a "two-dimensional surface") parallel to each layer. Hereinafter, the average value of the major axes of the two-dimensional surfaces may be referred to as "average flake size".

The larger the average flake size is, the better the orientation of MXene is in the material comprising MXene. The average value of the major axes of the two-dimensional surfaces is preferably 1.5 μm or more, and more preferably 2.5 μm or more. When the delamination treatment of MXene is performed by subjecting MXene to the ultrasonic treatment, most of MXene is reduced in a major axis to about several hundred nm by the ultrasonic treatment, and thus the film formed of the single-layer MXene delaminated by the ultrasonic treatment is considered to have low orientation of MXene.

The average value of the major axes of the two-dimensional surfaces is 20 μm or less, preferably 15 μm or less, and more preferably 10 μm or less from the viewpoint of dispersibility in the dispersion medium.

The major axis of the two-dimensional surface refers to a major axis when each MXene particle is approximated to an elliptical shape in an electron microscope photograph of MXene observed from a direction substantially orthogonal to a plane parallel to each layer, and the average value of the major axes of the two-dimensional surface refers to a number average of the major axes of 80 particles or more. As the electron microscope, a scanning electron microscope (SEM) photograph or a transmission electron microscope (TEM) photograph can be used.

The average value of the major axes of MXene of the present embodiment may be measured by dissolving a material comprising MXene in a solvent and dispersing the MXene in the solvent. Alternatively, it may be measured from an SEM image of the material.

(Average value of thickness of MXene)

The average value of the thickness of MXene in the present embodiment is preferably 1 nm to 100 $\mu$m. The thickness is preferably 50 $\mu$m or less, and more preferably 20 $\mu$m or less. On the other hand, in consideration of the thickness of the single-layer MXene particles, the lower limit of the thickness of MXene can be 1 nm.

The thickness of the MXene can be understood as a length in a direction substantially orthogonal to a plane parallel to each layer, and an average value of the thickness of the MXene is obtained as a number average dimension (for example, a number average of at least 40) based on an atomic force microscope (AFM) photograph or a transmission electron microscope (TEM) photograph.

The MXene described above can be produced by the following production method, but the MXene in the present disclosure is not limited to those produced by the following production method.

In one aspect, the method for producing MXene comprises:

(a) preparing a precursor represented by the following formula:

$$M_mAX_n$$

wherein M is at least one group 3, 4, 5, 6, and 7 metals,

X is a carbon atom, a nitrogen atom, or a combination thereof,
A is at least one group 12, 13, 14, 15, 16 element,
n is 1 to 4,
m is more than n and 5 or less;

(b) removing at least a part of the A atoms from the precursor by etching using an etching solution to obtain the etched product; and
(c) cleaning the etched product to obtain a cleaned product,
and may further comprises:
(d) performing an intercalation treatment on the etching treatment product in a dispersion medium using a metal-containing compound to obtain an intercalated product: and
(e) performing a delamination treatment on the intercalated product to obtain a delaminated product.

In one aspect, the etched product and the delaminated product can be used as the MXene, and preferably the cleaned product can be used as the MXene.

Each step is described below.

· Step (a)

First, a predetermined precursor is prepared. The predetermined precursor that can be used in the present embodiment is a MAX phase that is a precursor of MXene, and represented by the following formula:

$$M_mAX_n$$

wherein M is at least one group 3, 4, 5, 6, and 7 metals,

X is a carbon atom, a nitrogen atom, or a combination thereof,
A is at least one group 12, 13, 14, 15, 16 element,
n is 1 or more and 4 or less,
m is more than n and 5 or less.

M, X, n, and m have the same meaning as described above.

A is at least one group 12, 13, 14, 15, and 16 element, is usually a group A element, typically a group IIIA element and a group IVA element, and more particularly may comprise at least one selected from the group consisting of Al, Ga, In, Tl, Si, Ge, Sn, Pb, P, As, S, and Cd, and is preferably Al or Si.

The MAX phase has a crystal structure in which a layer constituted by A atoms is located between two layers represented by $M_mX_n$ (each X may have a crystal lattice located in an octahedral array of M). When typically m = n + 1, but not limited thereto, the MAX phase comprises repeating units in which each one layer of X atoms is disposed in between adjacent layers of n + 1 layers of M atoms (these are also collectively referred to as an "$M_mX_n$ layer"), and a layer of A atoms ("A atom layer") is disposed as a layer next to the (n + 1)th layer of M atoms.

The MAX phase can be produced by a known method. For example, a TiC powder, a Ti powder, and an Al powder are mixed in a ball mill, and the resulting mixed powder is fired under an Ar atmosphere to obtain a fired body (block-shaped MAX phase). Thereafter, the fired body obtained is pulverized by an end mill to obtain a powdery MAX phase for the next step.

· Step (b)

In the step (b), an etching treatment for removing at least a part of the A atoms from the precursor (MAX phase) represented by $M_mAX_n$ is performed. As a result, an etched product in which at least a part of the layer composed of A atoms is removed is obtained while the $M_mX_n$ layer in the precursor is maintained.

Conditions for the etching treatment are not particularly limited, and known conditions can be adopted. The etching may be performed using an etching solution comprising $F^-$. Such an etching solution may comprise hydrofluoric acid, hydrochloric acid, phosphoric acid, or the like as an acid. In one aspect, examples of the etching solution comprise hydrofluoric acid; a mixed solution of hydrofluoric acid and hydrochloric acid; and a mixed solution of lithium fluoride and hydrochloric acid, and all of them may further comprise phosphoric acid. As the solvent in the etching solution, water may be used, and for example, pure water may be used.

The etching treatment may be performed as a slurry by mixing the precursor and the etching solution.

In one aspect, in step (b), the intercalation treatment may be performed simultaneously. By allowing a metal-containing compound to be described later to coexist in the etching solution, the etching treatment and the intercalation treatment can be simultaneously performed. In this case, step (e) described later may be further performed.

When the intercalation treatment is simultaneously performed in the step (b), the content of the metal-containing compound in the total of the precursor, the metal-containing compound, and the etching solution can be, for example, 0.001% by mass to 10% by mass, further 0.01% by mass to 1% by mass, and particularly 0.1% by mass to 1% by mass.

· Step (c)

In the step (c), the treated product obtained by the etching treatment is cleaned to obtain a cleaned product. By performing the cleaning, the acid and the like used in the etching treatment can be sufficiently removed.

The cleaning may preferably be carried out with water. The amount of water mixed with the etched product and the cleaning method are not particularly limited. For example, stirring, centrifugation, and the like may be performed by adding water. Examples of the stirring method include a stirring method using a handshake, an automatic shaker, a share mixer, a pot mill, or the like. The degree of stirring such as the stirring speed and the stirring time may be adjusted according to the amount, concentration, and the like of the etched product to be treated. The cleaning with water may be performed once or more, and cleaning with water is preferably performed a plurality of times. For example, specifically, the cleaning with water may be performed by sequentially performing step (i) (to the treated product or the remaining precipitate obtained in the following (iii)), adding water and stirring, step (ii), centrifuging the stirred product, and step (iii) discarding the supernatant after centrifugation, and the steps (i) to (iii) may be repeated within a range of 2 times or more, for example, 15 times or less.

· Step (d)

In the step (d), an intercalation treatment for obtaining an intercalated product is performed by performing an intercalation treatment on the etched product in a dispersion medium using a metal-containing compound comprising a metal ion. As a result, an intercalated product in which a metal ion comprised in the metal-containing compound is intercalated between two adjacent $M_mX_n$ layers is obtained.

The metal ion may comprise a monovalent metal ion, and examples of the monovalent metal ion include alkali metal ions such as a lithium ion, a sodium ion, and a potassium ion, a copper ion, a silver ion, and a gold ion.

Examples of the metal-containing compound include an ionic compound in which the metal ion and the anion are bonded. Examples of the ionic compounds include a sulfide salt including an iodide, a phosphate, and a sulfate, a nitrate, an acetate, and a carboxylate of the above metal ion. As the metal ion, a lithium ion is preferable, and as the metal-containing compound, a metal-containing compound comprising a lithium ion is preferable, an ionic compound of a lithium ion is more preferable, and one or more of an iodide, a phosphate, and a sulfide salt of a lithium ion is further preferable. When a lithium ion is used as the metal ion, water hydrated to the lithium ion is considered to have the most negative dielectric constant, and thus it is easy to form a monolayer.

The content of the metal-containing compound in the total of the etched product, the metal-containing compound, and the dispersion medium can be, for example, 0.001% by mass to 10% by mass, further 0.01% by mass to 1% by mass, and particularly 0.1% by mass to 1% by mass. When the content of the metal-containing compound is within the above range, dispersibility in a dispersion medium is good.

A specific method of the intercalation treatment is not particularly limited, and for example, the dispersion medium, the etched product, and the metal-containing compound may be mixed and stirred, or may be left to stand. For example, stirring at room temperature can be mentioned. Examples of the stirring method include a method using a stirring bar such as a stirrer, a method using a stirring blade, a method using a mixer, a method using a centrifugal device, and the like, and the stirring time can be set according to the production scale of the single-layer/few-layer MXene particles, and can be set, for example, for 12 to 24 hours. The order of mixing the dispersion medium, the etched product, and the metal-containing compound is not particularly limited, but in one aspect, the dispersion medium and the etched product may be mixed, and then the metal-containing compound may be mixed. Typically, the etching solution after the etching treatment may be used as the dispersion medium.

· Step (e)

In the step (e), the intercalated product obtained by performing the intercalation treatment is subjected to a delamination treatment to obtain a delaminated product. The delamination treatment comprises peeling at least a part between two adjacent $M_m X_n$ layers by applying a shear stress to the intercalated product. By the delamination treatment, the MXene particles can be formed into a single layer or a small layer.

Conditions for the delamination treatment are not particularly limited, and the delamination treatment can be performed by a known method. For example, as a method of applying a shear stress to the intercalated product, there is a method of dispersing the intercalated product in a dispersion medium and stirring the dispersion medium. Examples of the stirring method include ultrasonic treatment, handshaking, and stirring using an automatic shaker. The degree of stirring such as the stirring speed and the stirring time may be adjusted according to the amount, concentration, and the like of the treated product to be treated. For example, the slurry after the intercalation is centrifuged to discard the supernatant liquid, then pure water is added to the remaining precipitate, and stirring is performed by, for example, a handshake or an automatic shaker to perform layer separation. The removal of the unpeeled substance comprises a step of performing centrifugal separation to discard the supernatant, and then cleaning the remaining precipitate with water. For example, (i) pure water is added to the remaining precipitate after discarding the supernatant and stirred, (ii) centrifugation is performed, and (iii) the supernatant is recovered. This operation of (i) to (iii) is repeated 1 time or more, preferably 2 times or more and 10 times or less to obtain a supernatant comprising single-layer/few-layer MXene particles as a delaminated product. Alternatively, the supernatant may be centrifuged, and the supernatant after centrifugation may be discarded to obtain a clay comprising single-layer/few-layer MXene particles as a delaminated product.

In the method for producing MXene, when the intercalation treatment is performed, the cleaning treatment may be further performed at an arbitrary stage after the intercalation treatment, preferably at a stage after the delamination treatment. By performing such a cleaning treatment, the metal ion and the metal-containing compound used for the intercalation can be sufficiently removed. Typically, such a cleaning treatment is performed after the step (e).

In one aspect, the cleaning treatment after the intercalation treatment may be performed in the same manner as in the step (c). In another aspect, after the delaminated product is acid-treated, the acid-treated product may be cleaned in the same manner as in the step (c). In these cases, the etched product in the step (c) may be replaced with a delaminated product or an acid-treated product, and the cleaning treatment may be performed.

The acid treatment can be performed by mixing and stirring the delaminated product and the acid solution. Examples of the acid include inorganic acids such as hydrochloric acid, sulfuric acid, nitric acid, phosphoric acid, perchloric acid, hydroiodic acid, hydrobromic acid, and hydrofluoric acid; an organic acid such as acetic acid, citric acid, oxalic acid, benzoic acid, or sorbic acid may be appropriately used, and the concentration of the acid in the acid solution may be appropriately adjusted according to the delaminated product. The stirring can be performed using a handshake, an automatic shaker, a share mixer, a pot mill, or the like. The acid treatment may be performed one or more times, and if necessary, an operation of mixing with a fresh acid solution (acid solution not used for the acid treatment) and stirring may be performed within a range of two or more times, for example, 10 times or less.

The intermediate and the target product in the production method described above may be isolated by a commonly used purification method. Examples the purification method includes suction filtration; and drying such as heat drying, freeze drying, and vacuum drying.

The MXene can have an action of improving the intestinal bacterial flora in vivo. In particular, in vivo, the production of intestinal bacteria can be promoted. Such intestinal bacteria may preferably comprise intestinal bacteria that increase a short-chain fatty acid (intestinal bacteria that metabolically produce a short-chain fatty acid). Intestinal bacteria that increase a short-chain fatty acid preferably comprise those of class Clostridia of phylum Firmicutes, more preferably those in family Eggerthellaceae of order Coriobacteriales of class Coriobacteriia of phylum Actinobacteriota, those in family Ruminococcaceae of order Oscillospirales of class Clostridia of phylum Firmicutes, those in family Lachnospiraceae of order Lachnospirales of class Clostridia of phylum Firmicutes, and even more preferably those in family Butyricicoccaceae of order Oscillospirales of class Clostridia of phylum Firmicutes.

Examples of the short-chain fatty acid include fatty acids having 1 to 4 carbon atoms, and preferably include butyric acid, acetic acid, and propionic acid. The short-chain fatty acid may have an action of repairing a blood vessel barrier. Such a blood vessel barrier comprises the intestinal barrier and the cerebral blood vessel barrier.

In addition, the MXene can have an action of lowering blood pressure in vivo.

Although the present disclosure should not be construed as being limited to a particular theory, the following (i) to (iii) are considered to be the actions of a short-chain fatty acid:

(i) repairing a blood vessel barrier;
(ii) enhancing anti-inflammatory action; and
(iii) ameliorating hypertension.

Regarding the above (i), a short-chain fatty acid contributes to maintenance of homeostasis of the blood barrier (In particular, the cerebral blood vessel endothelial cell/blood-brain barrier), suppresses excessive enhancement of permeability, and can act protectively on brain tissue. This can be confirmed by enhancement of Cldn, which is an RNA encoding Claudin, a protein forming the blood barrier (In particular, the blood-brain barrier). Such blood vessel barriers comprise the intestinal barrier and the blood-brain barrier.

Regarding the above (ii), a short-chain fatty acid is an action that acts as a ligand of a G-protein coupled receptor (GPR) and has an anti-inflammatory action via the immune system in the whole body. In the central nervous system, among a short-chain fatty acid, the permeation efficiency of the blood-brain barrier is particularly high, and has an anti-inflammatory action due to the inhibitory action of Histone Deacetylase (HDAC) activated at the injury site and the like. The action of butyric acid on the cerebral white matter can be confirmed, for example, from the fact that the GPR signaling pathway of the cerebral white matter is significantly enriched by MFT administration in RNA-sequencing data (functional enrichment analysis using Database for Annotation, Visualization and Integrated Discovery (DAVID)) of a hypertensive mouse.

Regarding the above (iii), a short-chain fatty acid may have an action of lowering blood pressure. Cerebral white matter (neural axons and myelin [complementary]) is a known fact to be damaged in hypertension. Such a hypotensive action can be confirmed from the viewpoint that a protective action against the central component (myelin) of the white matter is confirmed in the cerebral white matter of a hypertensive mouse, and the blood pressure decreases.

That is, MXene of the present disclosure may be used for one or two or more selected from the following (i) to (iii):

(i) repairing a blood vessel barrier;
(ii) enhancing anti-inflammatory action; and
(iii) ameliorating hypertension.

Ameliorating hypertension also comprises lowering of blood pressure in vivo.

The MXene can be used for treatment or prevention of a disease in which improvement of symptoms can be expected by repair of a blood vessel barrier in vivo. Examples of the disease include one or more selected from vascular dementia, Parkinson's disease, inflammatory bowel disease, chronic renal failure, irritable bowel syndrome, and ischemic and demyelinating central nervous system disease.

The MXene and the pharmaceutical composition can be administered orally.

The pharmaceutical composition according to the present embodiment can be in various dosage forms depending on usage. Examples of such a dosage form include powder, a granule, a fine granule, a dry syrup, a tablet, a capsule, liquid, and a sublingual agent, and also include an injection, an ointment, a suppository, and a patch.

The pharmaceutical composition according to the present embodiment can be a pharmaceutical composition comprising MXene as an active ingredient and further a pharmacologically acceptable additive by a publicly-known method according to the dosage form. Examples of such additives include an excipient, a disintegrant, a binder, a lubricant, a diluent, a buffering agent, an isotonizing agent, a preservative, a wetting agent, an emulsifying agent, a dispersing agent, a stabilizing agent, and a solubilizing agent. The pharmaceutical composition of the present disclosure can be prepared by appropriately mixing the MXene and the additive or diluting and dissolving the MXene with an additive.

The pharmaceutical composition according to the present embodiment can be administered systemically or locally, orally or parenterally (nasal, pulmonary, intravenous, enteral, subcutaneous, muscle, percutaneous). In one aspect, the pharmaceutical composition according to the present embodiment can be administered orally.

When the pharmaceutical composition of the present disclosure is used for treatment, the dose of MXene as an active ingredient thereof is appropriately determined depending on the age, sex, weight, disease, degree of treatment, and the like of the patient. For example, in the case of oral administration, the dose may be appropriately administered once or in several divided doses in the range of about 100 mg to 10 g/body per day of an adult (body weight of 60 kg) as an effective amount.

In addition, the pharmaceutical composition comprising MXene can be used for producing a medicine for treating or preventing a disease.

Examples

The present disclosure will be described more specifically with reference to the following examples, but the present disclosure is not limited thereto.

Example 1

[Production of MXene]

In Examples 1 and 2, (1) preparation of a precursor (MAX), (2) etching of the precursor, and (3) cleaning and drying described in detail below were sequentially performed to produce MXene two-dimensional particles.

(1) Production of precursor (MAX)

TiC powder, Ti powder, and Al powder (all manufactured by Kojundo Chemical Laboratory Co., Ltd.) were placed in a ball mill containing zirconia balls at a molar ratio of 2 : 1 : 1 and mixed for 24 hours. The obtained mixed powder was calcined in an Ar atmosphere at 1,350°C for 2 hours. The fired body (block) thus obtained was crushed with an end mill to a maximum size of 40 $\mu$m or less. In this way, $Ti_3AlC_2$ particles were obtained as a precursor (MAX).

(2) Etching of precursor

Using the $Ti_3AlC_2$ particles (powder) prepared by the above method, etching was performed under the following etching conditions to obtain a solid-liquid mixture (slurry) containing a solid component derived from the $Ti_3AlC_2$ powder.

(Etching conditions)

· Precursor: $Ti_3AlC_2$ (Sieving with 45 $\mu$m mesh)
· Etching solution composition: 50% by mass HF 6 mL,

    $H_2O$ 18 mL
    HCl (12M) 36 mL

· Precursor charge amount: 3.0 g
· Etching container: 100 mL Aiboy (wide-mouth bottle)
· Etching temperature: 35°C
· Etching time: 24 h
· Stirrer rotation speed: 400 rpm

(3) Cleaning and drying

The slurry was divided into two portions, each of which was inserted into two 50 mL centrifuge tubes, centrifuged under the condition of 3,500G using a centrifuge, and then the supernatant was discarded. An operation of adding 40 mL of pure water to each centrifuge tube, centrifuging again at 3,500 G, and separating and removing the supernatant was repeated 11 times. After final centrifugation, the supernatant was discarded to obtain a $Ti_3C_2T_s$-water medium clay. The obtained clay was dried by freeze drying to obtain dry powder of MXene.

Animal experiment 1 Administration experiment to hypertensive mice

Hypertension causes brain capillaries to become inflamed and produce chronic hypoperfusion. The symptom that the cognitive function is lowered by this is called vascular dementia. In animal experiment 2, MXene was administered to a mouse having genetically hypertension to remove inflammatory substances in the body, and it was confirmed whether recovery of cognitive function was expected. The administration period was 56 days, and the dose was 8% of the food consumption. After the end of the administration period, blood pressure fluctuation and cognitive behavior analysis (Novel Object Recognition Test) were performed. As a comparative example, a Ctl group to which MXene was not administered was also prepared.

(Blood pressure change)

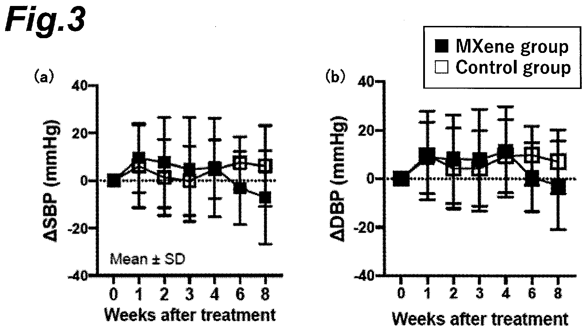

Changes in systolic blood pressure (SBP) and diastolic blood pressure (DBP) in the MXene group and the Ctl group are measured. Comparison was performed by taking a difference based on the blood pressure value on the first day. The results are shown in Figs. 3(a) and 3(b). A decrease in blood pressure was observed in the MXene group around the fourth week.

(Behavior analysis)

In the behavior analysis (Novel Object Recognition Test), a black box having a vertical width of 400 mm, a horizontal width of 400 mm, and a height of 400 mm was used. On days 1 and 2, mice were left in the box for 10 minutes to acclimatize. On day 3, two identical substances (old substances) were placed in cages, and the mice were left for 10 minutes. On day 4, one of the objects was changed to a new substance, and the mice were left for 10 minutes. Time for searching both objects was measured, and the ratio of the search time for a new substance to the total search time (Times of Entries Discrimination Index) was calculated according to the following equation:

$$\text{Times of Entries Discrimination Index} = [n/(n + f)] - 0.5$$

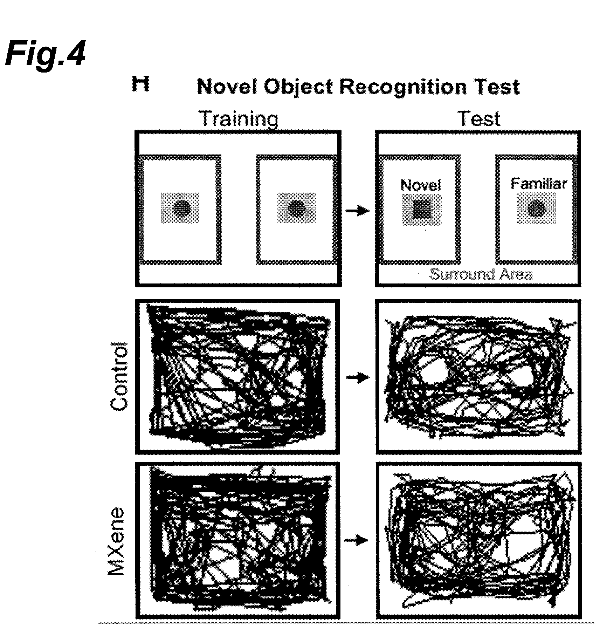

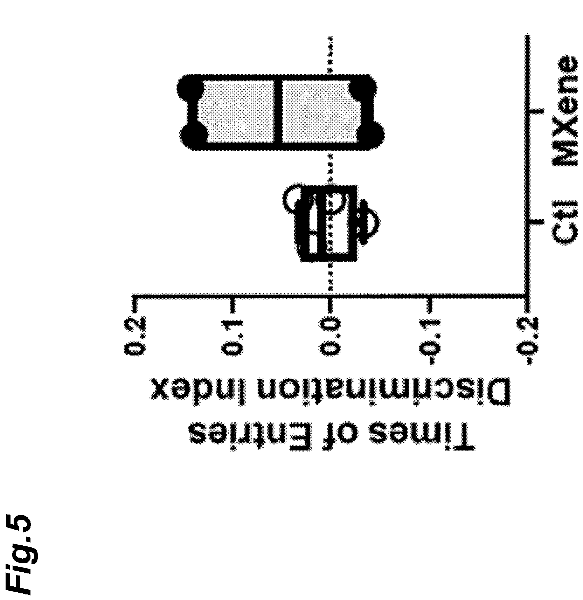

[In the formula, n represents the number of times of touching the new object, and f represents the number of times of touching the conventional object.]
and used as an index of memory learning ability. The mouse action region is shown in Fig. 4, and the Times of Entries Discrimination Index is shown in Fig. 5.
As shown in Fig. 5, in the Novel Object Recognition Test, the Times of Entries Discrimination Index was higher in the MXene group, and was a positive number. This indicates that the number of times of interest in and approaching the new substance is higher than that of the old substance. That is, it suggests that mouses recognized a new substance as something new, and that their cognitive function has recovered.

(Confirmation of intestinal bacterial flora)

As shown in Figs. 6(a) to 6(d), the abundance ratio of the intestinal bacterial flora of the mouse was compared between the groups by 16s-rRNA analysis. It was confirmed that those in family Eggerthellaceae of order Coriobacteriales of class Coriobacteriia of phylum Actinobacteriota, those in family Ruminococcaceae of order Oscillospirales of class Clostridia of phylum Firmicutes, those in family Lachnospiraceae of order Lachnospirales of class Clostridia of phylum Firmicutes, or those in family Butyricicoccaceae of order Oscillospirales of class Clostridia of phylum Firmicutes have proliferated.

(Confirmation of diversity of intestinal bacterial flora)

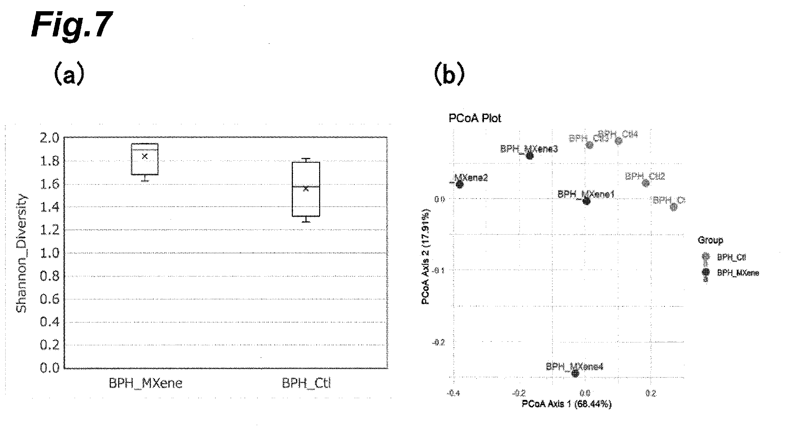

As shown in Figs. 7(a) and 7(b), the diversity of the intestinal bacterial flora of the mouse obtained by 16s-rRNA analysis was compared. The $\alpha$ diversity was visualized using the Shannon Index, and the $\beta$ diversity was visualized by PCoA (Principal Coordinates Analysis) after analysis based on the Bray-Curtis distance. It was confirmed that the diversity of the bacterial flora was changed between both groups by the administration of MXene.

(Confirmation of short-chain fatty acid)

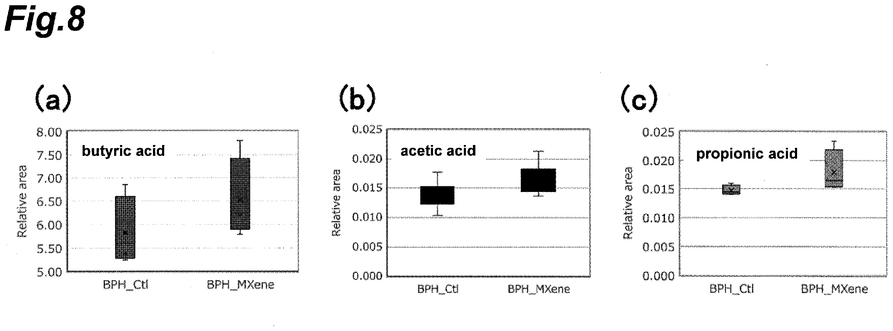

A metabolome analysis using CE-MS (capillary electrophoresis mass spectrometer) was performed on mouse serum, and changes in living body components by MXene were confirmed. As shown in Figs. 8(a) to 8(c), the proliferation of butyric acid, acetic acid, and propionic acid as a short-chain fatty acid was confirmed in the MXene group.

(Confirmation of RNA Change)

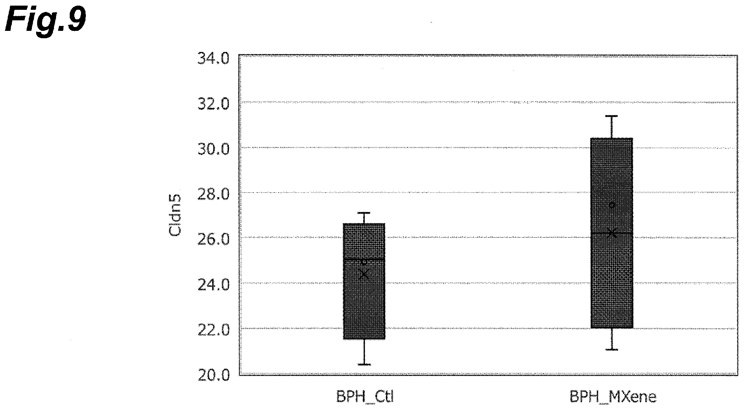

RNA_seq of a callosum cell was performed using a next generation sequencer. As shown in Fig. 9, proliferation of Cldn5, which is a gene encoded by a protein forming Blood Brain Barrier (Claudin), was confirmed.

(Confirmation of degree of brain tissue damage)

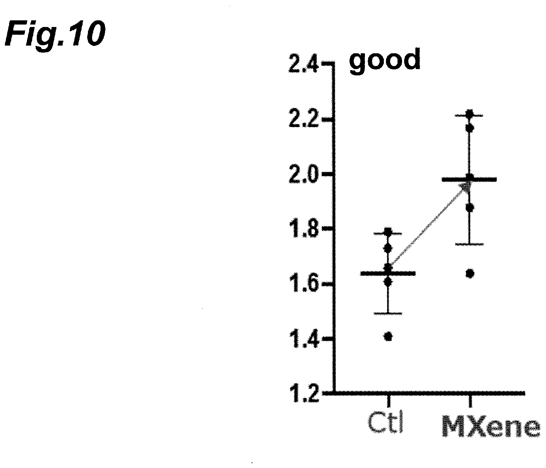

A Callosum staining evaluation of specimens was performed. The degree of damage can be confirmed by staining myelin that functionalizes nerve axons. The more myelin remains, the greater the brightness at the time of dyeing, and it can be determined that the damage is small. For image analysis, in order to correct variations at the time of imaging, the brightness of a white matter part based on a part called cortex was compared. As shown in Fig. 10, it was confirmed that the brightness was significantly higher and white matter damage was suppressed in the MXene group.

In response to the above results, WB measurement of MBP was performed in order to measure the amount of myelin. The method was a general WB method, in which a protein was extracted from a tissue of callosum, transferred to a membrane after gel electrophoresis, and a band was detected by an antibody reaction. In the measurement, the amount of MBP based on the amount of $\beta$-actin as the total amount of protein was compared among the specimens.

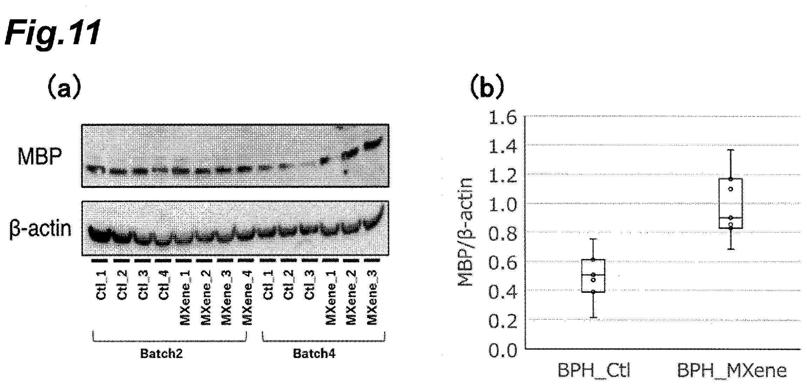

Quantification by image processing was performed based on the marker floating on the membrane. As shown in Figs. 11(a) and 11(b), an increase in MBP was confirmed in the MXene administration group.

It was observed that MXene increases the short-chain fatty acid-producing bacteria, those in family Eggerthellaceae of order Coriobacteriales of class Coriobacteriia of phylum Actinobacteriota, those in family Ruminococcaceae of order Oscillospirales of class Clostridia of phylum Firmicutes, those in family Lachnospiraceae of order Lachnospirales of class Clostridia of phylum Firmicutes, or those in family Butyricicoccaceae of order Oscillospirales of class Clostridia of phylum Firmicutes in the intestine. The proliferated short-chain fatty acid is considered to have promoted the decrease in blood pressure and the repair of BloodBrainBarrier, and contributed to the improvement of the cognitive function.

Furthermore, in Examples, it was confirmed that MXene conditions intestinal bacteria and proliferates short-chain fatty acid (SCFAs)-producing bacteria, particularly butyric acid-producing bacteria. Here, there are roughly three conceivable actions of SCFAs (particularly butyric acid).

The first is to contribute to the maintenance of homeostasis of the cerebral blood vessel endothelial cell/blood-brain barrier, suppress excessive enhancement of permeability, and act on brain tissue in a protective manner. This can be confirmed by enhancement of Cldn5 that is an RNA encoding Claudin that is a protein forming the blood-brain barrier.

The second is an action that acts as a ligand of a G-protein coupled receptor (GPR) and has an anti-inflammatory action via the immune system in the whole body. In the central nervous system, among a short-chain fatty acid, the permeation efficiency of the blood-brain barrier is particularly high, and has an anti-inflammatory action due to the inhibitory action of Histone Deacetylase (HDAC) activated at the injury site and the like. In fact, in the above Examples, also in the RNA-sequencing data (functional enrichment analysis using Database for Annotation, Visualization and Integrated Discovery (DAVID)) of the hypertensive mouse, the GPR signaling pathway of the cerebral white matter was observed to have been significantly enriched by MFT administration, and butyric acid acts on the cerebral white matter.

The third is an action of lowering blood pressure. Cerebral white matter (neural axons and myelin (complementary)) is a known fact to be damaged in hypertension. Therefore, in the cerebral white matter of a hypertensive mouse, it is supposedly confirmed that Mxene have a hypotensive action based on the observed actions; the protective action on the central component (myelin) of the white matter and the lowered blood pressure.

From the above points, it was confirmed that MXene has a multi-target protective action against damaged white matter by conditioning intestinal bacteria and proliferating short-chain fatty acid (SCFAs)-producing bacteria, particularly butyric acid-producing bacteria.

The present disclosure comprises the following:

[1] A pharmaceutical composition comprising MXene for improving intestinal bacterial flora in vivo or increasing a short-chain fatty acid in vivo.

[2] The pharmaceutical composition according to [1], wherein

the Mxene comprises two-dimensional particles having one or more layers, and
the layer comprises at least one metal selected from group 3, 4, 5, 6, and 7 metals and at least one selected from a carbon atom and a nitrogen atom.

[3] The pharmaceutical composition according to [2], wherein the layer comprises a layer body represented by the following formula:

$$M_mX_n$$

wherein M is at least one group 3, 4, 5, 6, and 7 metals,

X is a carbon atom, a nitrogen atom, or a combination thereof,
n is 1 to 4, and
m is more than n and 5 or less.

[4] The pharmaceutical composition according to [3], wherein the layer further comprises a modification or termination T present on a surface of the layer body in which T is at least one selected from the group consisting of a hydroxyl group, a fluorine atom, a chlorine atom, an oxygen atom, and a hydrogen atom.
[5] The pharmaceutical composition according to any one of [1] to [4], wherein the Mxene comprises $Ti_3C_2$.
[6] The pharmaceutical composition according to any one of [1] to [5], wherein the improving intestinal bacterial flora comprises proliferating intestinal bacteria that increase a short-chain fatty acid.
[7] The pharmaceutical composition according to any one of [1] to [6], wherein intestinal bacteria metabolizing the short-chain fatty acid comprise those in class Clostridia of phylum Firmicutes.
[8] The pharmaceutical composition according to any one of [1] to [7], wherein intestinal bacteria metabolizing the short-chain fatty acid comprise those in family Eggerthellaceae of order Coriobacteriales of class Coriobacteriia of phylum Actinobacteriota, those in family Ruminococcaceae of order Oscillospirales of class Clostridia of phylum Firmicutes, those in family Lachnospiraceae of order Lachnospirales of class Clostridia of phylum Firmicutes, or those in family Butyricicoccaceae of order Oscillospirales of class Clostridia of phylum Firmicutes.
[9] The pharmaceutical composition according to any one of [1] to [8], wherein the short-chain fatty acid comprises butyric acid.
[10] The pharmaceutical composition according to any one of [1] to [9], which is further used for one or two or more selected from the following (i) to (iii):

(i) repairing a blood vessel barrier;
(ii) enhancing anti-inflammatory action; and
(iii) ameliorating hypertension.

[11] The pharmaceutical composition according to any one of [1] to [10], wherein the blood vessel barrier is an intestinal barrier or a blood-brain barrier.
[12] The pharmaceutical composition according to any one of [1] to [11], which is further used for lowering blood pressure.
[13] The pharmaceutical composition according to any one of [1] to [12], which is for oral administration.
[14] A method for improving intestinal bacterial flora or increasing a short-chain fatty acid in vivo, comprising administering an effective amount of MXene to a subject.
[15] The method according to [14], wherein the MXene is administered orally.
[16] A method for treating or preventing a disease in which improvement of symptoms can be expected by repair of a blood vessel barrier,
the method comprising administering an effective amount of MXene to a subject.
[17] The method according to [16], wherein the disease comprises one or more selected from vascular dementia, Parkinson's disease, inflammatory bowel disease, chronic renal failure, irritable bowel syndrome, and ischemic and demyelinating central nervous system disease.
[18] The method for treating or preventing according to [16], wherein the MXene is administered orally.

REFERENCE SIGNS LIST

1a, 1b Layer body ($M_mX_n$ layer)
3a, 5a, 3b, 5b Modifier or terminal T
7a, 7b MXene layer
10, 10a, 10b MXene particles (two-dimensional particles of layered material)

**Claims**

1. A pharmaceutical composition comprising MXene for improving intestinal bacterial flora in vivo or increasing a short-chain fatty acid in vivo.

2. The pharmaceutical composition according to claim 1, wherein

    the Mxene comprises two-dimensional particles having one or more layers, and
    the layer comprises at least one metal selected from group 3, 4, 5, 6, and 7 metals and at least one selected from a carbon atom and a nitrogen atom.

3. The pharmaceutical composition according to any one of claims 1 or 2, wherein the improving intestinal bacterial flora comprises proliferating intestinal bacteria that increase a short-chain fatty acid.

4. The pharmaceutical composition according to any one of claims 1 to 3, wherein intestinal bacteria metabolizing the short-chain fatty acid comprise those in class Clostridia of phylum Firmicutes.

5. The pharmaceutical composition according to any one of claims 1 to 74, wherein intestinal bacteria metabolizing the short-chain fatty acid comprise those in family Eggerthellaceae of order Coriobacteriales of class Coriobacteriia of phylum Actinobacteriota, those in family Ruminococcaceae of order Oscillospirales of class Clostridia of phylum Firmicutes, those in family Lachnospiraceae of order Lachnospirales of class Clostridia of phylum Firmicutes, or those in family Butyricicoccaceae of order Oscillospirales of class Clostridia of phylum Firmicutes.

6. The pharmaceutical composition according to any one of claims 1 to 5, wherein the short-chain fatty acid comprises butyric acid.

7. The pharmaceutical composition according to any one of claims 1 to 6, which is further used for one or two or more selected from the following (i) to (iii):

    (i) repairing a blood vessel barrier;
    (ii) enhancing anti-inflammatory action; and
    (iii) ameliorating hypertension.

8. The pharmaceutical composition according to any one of claims 1 to 7, wherein the blood vessel barrier is an intestinal barrier or a blood-brain barrier.

9. The pharmaceutical composition according to any one of claims 1 to 8, which is further used for lowering blood pressure.

10. The pharmaceutical composition according to any one of claims 1 to 9, which is for oral administration.

11. A method for improving intestinal bacterial flora or increasing a short-chain fatty acid in vivo, comprising administering an effective amount of MXene to a subject.

12. The method according to claim 14, wherein the MXene is administered orally.

13. A method for treating or preventing a disease in which improvement of symptoms can be expected by repair of a blood vessel barrier,
    the method comprising administering an effective amount of MXene to a subject.

14. The method according to claim 16, wherein the disease comprises one or more selected from vascular dementia, Parkinson's disease, inflammatory bowel disease, chronic renal failure, irritable bowel syndrome, and ischemic and demyelinating central nervous system disease.

15. The method for treating or preventing according to claim 13 or 14, wherein the MXene is administered orally.

*Fig.1 (a)*

*Fig.1 (b)*

EP 4 585 218 A1

**Fig.2**

Fig.3

Fig.4

Fig.5

EP 4 585 218 A1

# Fig.6

(a)

(b)

(c)

(d)

Eggerthellaceae

Ruminococcaceae

Lachnospiraceae

Butyricicoccaceae

**Fig.7**

(a)

(b)

EP 4 585 218 A1

**Fig.8**

(a)

(b)

(c)

*Fig.9*

**Fig.10**

# Fig.11

## (a)

MBP

β-actin

Ctl_1 Ctl_2 Ctl_3 Ctl_4 MXene_1 MXene_2 MXene_3 MXene_4 Ctl_1 Ctl_2 Ctl_3 MXene_1 MXene_2 MXene_3

Batch2　　　Batch4

## (b)

MBP/β-actin

BPH_Ctl　　BPH_MXene

Europäisches
Patentamt

European
Patent Office

Office européen
des brevets

# EUROPEAN SEARCH REPORT

**Application Number**

EP 25 15 1303

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 115 487 310 A (XIANGYA HOSPITAL CENTRAL SOUTH UNIV) 20 December 2022 (2022-12-20) * the whole document * | 1-8, 10-15 | INV. A61K33/00 A61K33/24 A61P1/00 A61P9/10 A61P9/12 A61P29/00 |
| X | XIANGPING SONG ET AL: "Efficient Therapy of Inflammatory Bowel Disease (IBD) with Highly Specific and Durable Targeted Ta2C Modified with Chondroitin Sulfate (TACS)", ADVANCED MATERIALS, VCH PUBLISHERS, DE, vol. 35, no. 36, 23 July 2023 (2023-07-23) , page n/a, XP072540146, ISSN: 0935-9648, DOI: 10.1002/ADMA.202301585 * the whole document * | 1-8, 10-15 | |
| X | HOU LINQIAN ET AL: "Orally administered titanium carbide nanosheets as anti-inflammatory therapy for colitis", THERANOSTICS, vol. 12, no. 8, 1 January 2022 (2022-01-01), pages 3834-3846, XP093100691, AU ISSN: 1838-7640, DOI: 10.7150/thno.70668 * the whole document * | 13-15 | |
| X | WO 2023/204278 A1 (MURATA MANUFACTURING CO [JP]) 26 October 2023 (2023-10-26) * the whole document * | 13-15 | |
| A | WO 2024/007070 A1 (UNIV MANITOBA [CA]) 11 January 2024 (2024-01-11) * abstract; claims; examples * | 1-15 | |

**TECHNICAL FIELDS SEARCHED (IPC)**

A61K
A61P

-/--

The present search report has been drawn up for all claims

| Place of search | Date of completion of the search | Examiner |
|---|---|---|
| The Hague | 1 May 2025 | Hoff, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

**EP 4 585 218 A1**

Europäisches
Patentamt

European
Patent Office

Office européen
des brevets

## EUROPEAN SEARCH REPORT

**Application Number**

EP 25 15 1303

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | "Thematic poster session (TPS)", ALLERGY, WILEY-BLACKWELL PUBLISHING LTD, UNITED KINGDOM, vol. 79, 25 October 2024 (2024-10-25), pages 358-904, XP072725051, ISSN: 0105-4538, DOI: 10.1111/ALL.16300 * page 522 * * abstract no.000137 * | 1-15 | |
| X,P | JIFENG YU ET AL: "Probiotics Bi-Enzymatic Cascade Repair System for Editing the Inflammatory Microenvironment to Boost Probiotic Therapy in Inflammatory Bowel Disease", ADVANCED MATERIALS, VCH PUBLISHERS, DE, vol. 37, no. 4, 6 December 2024 (2024-12-06), page n/a, XP072820738, ISSN: 0935-9648, DOI: 10.1002/ADMA.202412429 * the whole document * | 1-15 | |

| | |
|---|---|
| | **TECHNICAL FIELDS SEARCHED (IPC)** |

The present search report has been drawn up for all claims

| Place of search | Date of completion of the search | Examiner |
|---|---|---|
| The Hague | 1 May 2025 | Hoff, Philippe |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...............................................................................
& : member of the same patent family, corresponding
    document

EPO FORM 1503 03.82 (P04C01)

page 2 of 2

27

**ANNEX TO THE EUROPEAN SEARCH REPORT
ON EUROPEAN PATENT APPLICATION NO.**

EP 25 15 1303

This annex lists the patent family members relating to the patent documents cited in the above-mentioned European search report.
The members are as contained in the European Patent Office EDP file on
The European Patent Office is in no way liable for these particulars which are merely given for the purpose of information.

01-05-2025

| Patent document cited in search report | | Publication date | Patent family member(s) | | Publication date |
|---|---|---|---|---|---|
| CN 115487310 | A | 20-12-2022 | NONE | | |
| WO 2023204278 | A1 | 26-10-2023 | CN | 119031922 A | 26-11-2024 |
| | | | CN | 119031979 A | 26-11-2024 |
| | | | EP | 4491187 A1 | 15-01-2025 |
| | | | JP | WO2023204278 A1 | 26-10-2023 |
| | | | JP | WO2023204279 A1 | 26-10-2023 |
| | | | US | 2025032694 A1 | 30-01-2025 |
| | | | US | 2025041331 A1 | 06-02-2025 |
| | | | WO | 2023204278 A1 | 26-10-2023 |
| | | | WO | 2023204279 A1 | 26-10-2023 |
| WO 2024007070 | A1 | 11-01-2024 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023012558 A

**Non-patent literature cited in the description**

- **LIFU WANG et al.** An engineered probiotic secreting Sj16 ameliorates colitis via Ruminococcaceae/butyrate/retinoic acid axis. *BIOENGINEERING & TRANSLATIONAL MEDICINE*, September 2021, vol. 6 (3)
- **V. BRANISTE et al.** The gut microbiota influences blood-brain barrier permeability in mice. *Science Translational Meddicine*, 2014, vol. 6, 263ra158
- **LUISA F. GOMEZ-ARANGO et al.** Increased Systolic and Diastolic Blood Pressure Is Associated With Altered Gut Microbiota Composition and Butyrate Production in Early Pregnancy. *Hyperteinsion*, October 2016, vol. 68 (4)